# FASCICULE DE BREVET EUROPEEN

(11) **EP 3 917 597 B1**
(45) Date de publication et mention de la délivrance du brevet: **09.07.2025**
(21) Numéro de dépôt: 20707719.9
(22) Date de dépôt: 31.01.2020
(51) Int. Cl.: A61M 5/20, A61M 5/30

(54) **DISPOSITIF DE CONTRÔLE DE PRESSION OU ALTERNATIVEMENT DE FORCE D'INJECTION D'UNE PLURALITÉ DE JETS GÉNÉRÉS EN SORTIE D'UN DISPOSITIF D'INJECTION SANS AIGUILLE**
VORRICHTUNG ZUR STEUERUNG DES DRUCKS ODER ALTERNATIV DER INJEKTIONSKRAFT VON MEHREREN AM AUSLASS EINER NADELLOSEN INJEKTIONSVORRICHTUNG ERZEUGTEN STRAHLEN
DEVICE FOR CONTROLLING THE PRESSURE OR ALTERNATIVELY THE INJECTION FORCE OF A PLURALITY OF JETS GENERATED AT THE OUTLET OF A NEEDLELESS INJECTION DEVICE

(30) Priorité: 01.02.2019 FR 1900997
(43) Date de publication de la demande: 08.12.2021
(73) Titulaire: Crossject, 21000 Dijon (FR)
(72) Inventeur: AURIEL, Christophe, 21270 Binges (FR); JAUMARY, Ludovic, 21000 Dijon (FR); ROBIN, Maxime, 69270 Fontaines Saint Martin (FR); SIMONNEAU, Brice, 69100 Villeurbanne (FR)
(74) Mandataire: Germain Maureau
(86) Numéro de dépôt international: PCT/FR2020/050158
(87) Numéro de publication internationale: WO 2020/157443

(56) Documents cités:
- WO-A1-2018/069600
- CN-B- 103 323 174
- JP-A- 2005 021 640

## Description

L'invention se rapporte à un dispositif de contrôle de pression ou alternativement de force d'injection d'une pluralité de jets générés par des canaux en sortie d'une buse d'un dispositif d'injection sans aiguille.

On connaît des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif à usage thérapeutique en médecine humaine ou vétérinaire.

Le principe actif est constitué par un liquide pouvant être visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié.

Un tel dispositif d'injection comporte de manière connue, comme par exemple dans la demande de brevet FR 2 815 544 A, un corps comprenant successivement un générateur de gaz, une chambre d'expansion, un réservoir contenant le principe actif et un système d'injection.

Le réservoir est inséré dans un logement tubulaire du corps du dispositif d'injection, en étant obturé par un piston supérieur, ou amont, et un piston inférieur, ou aval. L'extrémité libre inférieure du réservoir coopère avec le système d'injection comprenant une buse d'injection comportant plusieurs canaux d'injection s'étendant axialement suivant un axe d'injection. Le diamètre des canaux d'injection est compatible avec la granulométrie du principe actif pour éviter qu'il soit obturé.

Pour permettre l'injection du principe actif, le corps est monté coulissant dans un capot creux enveloppant le corps, de bas en haut suivant un axe de coulissement, entre une position de repos et une position d'injection, l'entraînement du corps étant réalisé lorsque l'utilisateur appui la buse d'injection sur sa peau. Le déplacement du corps dans le capot permet le déclenchement du générateur de gaz, générant un gaz sous pression qui entraîne en déplacement les pistons pour injecter le principe actif à travers la peau du patient, en passant par la buse d'injection.

Dans cette position d'injection, le principe actif est éjecté depuis les canaux de la buse d'injection, dans une direction d'injection, selon des jets ayant une pression d'injection prédéterminée permettant au principe actif de traverser la peau du patient à la profondeur souhaitée pour assurer une injection optimale de celui-ci. La pression d'injection de ces jets est donc déterminante de la profondeur d'injection dans la peau du principe actif injecté.

Par ailleurs, pour permettre une injection réussie, les jets en sortie de la buse d'injection doivent être délivrés selon cette pression d'injection pendant un temps d'injection prédéterminé garantissant l'injection de la quantité de principe actif désirée.

Ces grandeurs de pression ou alternativement de force d'injection et de temps d'injection sont, bien entendu, dépendantes du principe actif à injecter.

Il existe donc un besoin pour un dispositif de contrôle de la pression ou alternativement de la force d'injection des jets générés par un dispositif d'injection sans aiguille, qui permettent une mesure fidèle de la pression ou alternativement de la force d'injection de ces jets. Un dispositif de contrôle est connu de JP 2005021640 A, ce dispositif ne contrôle toutefois qu'un seul jet.

A cet effet, l'invention a pour objet un dispositif de contrôle de pression ou alternativement de la force d'injection d'une pluralité de jets générés par des canaux en sortie d'une buse d'un dispositif d'injection sans aiguille. Le dispositif de contrôle de pression ou alternativement de force selon l'invention est remarquable en ce qu'il comprend pour chaque jet généré en sortie de la buse un élément de mesure de pression ou alternativement de force d'injection du jet correspondant, chaque élément de mesure de pression ou alternativement de la force d'injection comprenant une face de réception du jet correspondant orientée pour recevoir ce jet.

La mesure indépendante de la pression ou alternativement de la force d'injection de chacun de ces jets permet avantageusement d'identifier la défaillance d'un canal d'injection d'une buse ne délivrant pas un jet selon une pression ou alternativement une force d'injection prédéterminée. L'identification d'une telle défaillance a pour but de garantir la conformité des dispositifs d'injection sans aiguille lors du contrôle d'un lot de fabrication par exemple.

Selon un mode de réalisation de l'invention, au moins la face de réception d'un élément de mesure de pression ou alternativement de force d'injection est inclinée par rapport à un plan orthogonal à la direction d'injection du jet correspondant.

L'inclinaison de la face de réception d'un élément de mesure de pression ou alternativement de force d'injection permet de limiter l'incidence d'un jet voisin sur la face de réception de cet élément de mesure de pression ou alternativement de force d'injection.

Avantageusement, la face de réception de chaque élément de mesure de pression ou alternativement de force d'injection est inclinée par rapport à un plan orthogonal à la direction d'injection du jet correspondant.

Avantageusement, l'inclinaison de chaque face de réception est orientée distinctement l'une de l'autre.

Selon un mode de réalisation de l'invention, l'inclinaison de la face de réception d'au moins un élément de mesure de pression ou alternativement de force d'injection est obtenue par l'inclinaison dans sa longueur de l'élément de mesure de pression ou alternativement de force d'injection correspondant par rapport audit plan orthogonal à la direction d'injection.

Avantageusement, l'inclinaison de la face de réception de chaque élément de mesure de pression ou alternativement de force d'injection est obtenue par l'inclinaison dans sa longueur de l'élément de mesure de pression ou alternativement de force d'injection correspondant par rapport à la direction d'injection.

Selon un autre mode de réalisation de l'invention, au moins un élément de mesure de pression ou alternativement de force d'injection est formé par l'assemblage d'un capteur de pression ou alternativement de force d'injection et d'une pièce rapportée comprenant la face de réception du jet correspondant.

La pièce rapportée comprend avantageusement un élément de transmission mécanique en contact avec l'élément de mesure de pression. L'élément de transmission mécanique permet avantageusement de transmettre les vibrations mécaniques reçues par la pièce rapportée à l'élément de mesure de pression.

De façon avantageuse, la pièce rapportée est détachable par rapport à l'élément de mesure de pression.

Avantageusement, la face de réception est une face en biseau de la pièce rapportée.

Dans cette dernière configuration, pour chaque élément de mesure de pression ou alternativement de force d'injection, le capteur de pression ou alternativement de force s'étend dans sa longueur parallèlement à la direction d'injection du jet correspondant.

On comprendra que chaque capteur s'étend selon une direction coaxiale à l'axe d'un canal de sortie par lequel est projeté le jet correspondant.

Selon une variante de réalisation de l'invention, le dispositif de contrôle de pression ou alternativement de force comprend un support portant solidairement chacun des éléments de mesure de pression ou alternativement de force d'injection.

Le support peut avantageusement comprendre des ergots d'indexage des éléments de mesure de pression ou alternativement de force d'injection.

Ces ergots d'indexage permettent avantageusement l'indexage radial et axial des pièces rapportées.

Lorsqu'une pièce rapportée est détachable par rapport à l'élément de mesure de pression, on comprendra qu'elle peut être limitée par les ergots d'indexage comprenant des doigts d'indexage prévue pour le maintien de la pièce rapportée.

Selon d'autres variantes de réalisation, une pièce rapportée peut être solidaire de l'élément de mesure de pression par des modifications structurelles telles que : l'ajout d'un élément de fixation liquide ou visqueux comme de la cire, de la colle, de la résine par exemple ou par fixation mécanique telle que le sertissage.

Selon une autre variante de réalisation de l'invention, le dispositif de contrôle de pression ou alternativement de force comprend un organe de cloisonnement des jets les uns par rapport aux autres.

L'organe de cloisonnement permet d'empêcher l'incidence d'un jet voisin sur la face de réception d'un élément de mesure correspondant.

L'organe de cloisonnement des jets peut avantageusement comprendre des séparateurs délimitant les éléments de mesure de pression ou alternativement de force d'injection les uns des autres en vue d'empêcher l'incidence d'un jet voisin sur la face de réception d'un élément de mesure de pression ou alternativement de force d'injection correspondant.

Selon une autre variante de réalisation de l'invention, le dispositif de contrôle de pression ou alternativement de force comprend un élément d'indexage des canaux de la buse par rapport aux éléments de mesure de pression ou alternativement de force d'injection.

L'élément d'indexage peut avantageusement comprendre une base de réception de la buse et au moins deux points d'indexage prévus pour assurer la mise en position de l'élément d'indexage par rapport aux éléments de mesure de pression ou alternativement de force d'injection.

Selon une autre variante de réalisation de l'invention, le dispositif de contrôle de pression ou alternativement de force est composé d'un capteur de force ou de pression et d'une interface.

L'interface permet l'acquisition et le traitement des signaux électriques transmis par les éléments de mesure de pression ou alternativement de force d'injection.

L'invention concerne par ailleurs un ensemble de contrôle de pression ou alternativement de force d'injection comprenant un dispositif d'injection sans aiguille et un dispositif de contrôle de pression ou alternativement de force d'injection tel que défini dans le présent document.

Selon un mode de réalisation de cet ensemble, le dispositif d'injection sans aiguille comporte un corps comprenant successivement un générateur de gaz, une chambre d'expansion, un réservoir contenant un principe actif et un système d'injection, le système d'injection comprenant la buse d'injection.

Avantageusement, le corps est monté coulissant dans un capot creux enveloppant le corps, suivant un axe de coulissement, entre une position de repos et une position d'injection.

Le principe actif contenu dans le réservoir peut avantageusement être choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Méthylnaltrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medroxyprogesterone acetate,
- Medroparin calcium,
- Methylprednisolone acetate
- Heparin calcium,
- Terbutaline.

L'invention concerne par ailleurs un procédé de contrôle de pression ou alternativement de force d'injection d'une pluralité de jets générés par des canaux en sortie d'une buse d'un dispositif d'injection sans aiguille, le procédé étant réalisé à l'aide d'un dispositif de contrôle de pression ou alternativement de force tel que défini dans le présent document,

Le procédé de contrôle est remarquable en ce qu'il comprend :
- une étape d'indexage dans laquelle les canaux de la buse du dispositif d'injection sans aiguille sont mis en regard des éléments de mesure de pression ou alternativement de force d'injection dudit dispositif de contrôle ;
- une étape de mesure de pression ou alternativement de force d'injection dans laquelle chacun des jets est mesuré indépendamment les uns des autres.

D'autres aspects, buts et avantages de l'invention apparaîtront à la lecture de la description détaillée suivante de formes de réalisation préférées de celle-ci, donnée à titre d'exemple non limitatif et faite en référence aux dessins annexés sur lesquels :
[Fig. 1] illustre une vue en perspective d'un dispositif de contrôle de pression ou alternativement de force d'injection selon un premier mode de réalisation de l'invention qui est mis en correspondance d'un dispositif d'injection sans aiguille,
[Fig. 2] illustre une vue détaillée du premier mode de réalisation du dispositif de contrôle de pression ou alternativement de force représenté à la figure 1,
[Fig. 3] illustre le dispositif de contrôle de pression ou alternativement de force de la figure 1 reçu dans un socle d'un poste opérationnel,
[Fig. 4] illustre une vue en perspective d'un deuxième mode de réalisation de l'invention du dispositif de contrôle,
[Fig. 5] illustre une vue en perspective d'un troisième mode de réalisation de l'invention et comprenant un organe de cloisonnement des jets du dispositif de contrôle,
[Fig. 6] illustre une vue en perspective d'un élément d'indexage du dispositif d'injection sans aiguille par rapport au dispositif de contrôle de pression ou alternativement de force d'injection,
[Fig. 7] illustre une vue détaillée de l'élément d'indexage de la figure 6.

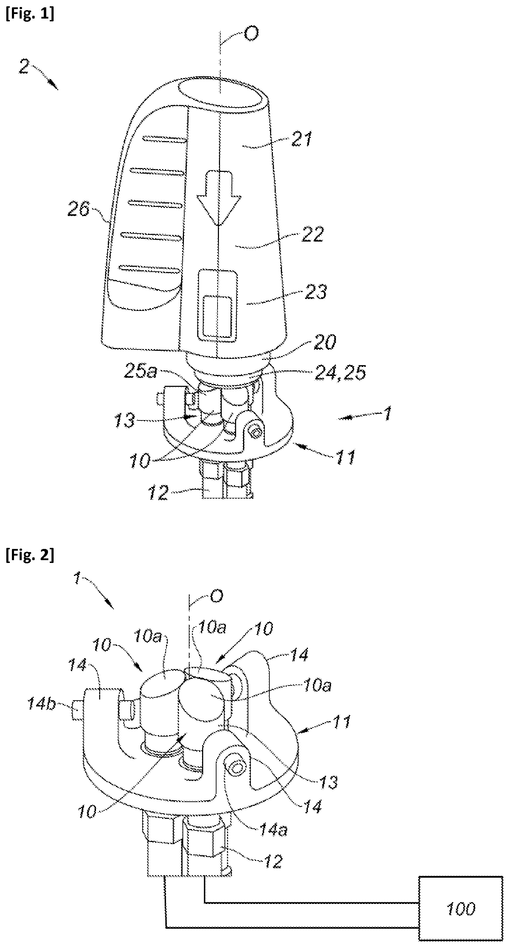

Aux figures 1 à 3, on a représenté un dispositif de contrôle de pression ou alternativement de force d'injection 1 selon un premier mode de réalisation de l'invention. Ce dispositif de contrôle de pression ou alternativement de force d'injection 1 est prévu pour mesurer la pression ou alternativement la force d'injection de chacun des jets générés ou éjectes depuis une buse d'injection 25 d'un dispositif d'injection sans aiguille 2 représenté à la figure 1. Le dispositif d'injection sans aiguille 2 et le dispositif de contrôle de pression ou alternativement de force d'injection 1 forment un ensemble de contrôle de pression ou alternativement de force d'injection dédié à cette mesure.

Le dispositif d'injection sans aiguille 2 représenté, ou seringue sans aiguille, comporte un corps 20 comprenant successivement un générateur de gaz 21, une chambre d'expansion 22, un réservoir 23 actionné par des pistons amont et aval et contenant un principe actif et un système d'injection 24 du principe actif.

Le système d'injection 24 comprend la buse d'injection 25 présentant une forme cylindrique autour d'un axe principal ou direction d'injection O. La buse 25 est prévue pour coopérer le corps 20. La buse 25 comprend au moins un canal 25a de sortie, ici trois canaux, parallèles à l'axe principal d'injection O.

Lorsque la buse d'injection 25 est appuyée, le corps 20 coulisse dans un capot creux 26 l'enveloppant entre une position de repos et une position d'injection. Le déplacement du corps 20 dans le capot 26 permet le déclenchement du générateur de gaz 21, générant un gaz sous pression qui entraîne en déplacement les pistons pour éjecter le principe actif depuis la buse d'injection 25.

La direction de propagation de ces jets est parallèle à la direction d'injection O du système d'injection 24.

Afin de mesurer la pression ou la force de chacun de ces jets, la buse d'injection 25 du dispositif d'injection sans aiguille 2 est disposée en regard du dispositif de contrôle de pression ou alternativement de force d'injection 1.

Le dispositif de contrôle de pression ou alternativement de force d'injection 1 comporte, pour chaque jet généré par la buse 25 du dispositif d'injection sans aiguille 2, un élément de mesure 10 de pression ou alternativement de force d'injection dédié à sa mesure. On comprendra alors que la mesure de chaque jet est réalisée individuellement.

Chacun élément de mesure 10 de pression ou alternativement de force d'injection est prévu pour être relié à une unité de traitement 100 réalisant l'acquisition des signaux électriques transmis par les éléments de mesure 10 de pression ou alternativement de force d'injection. Chaque élément de mesure 10 est avantageusement connecté à l'unité de traitement 100 par son extrémité libre.

Ces signaux sont préférentiellement traités indépendamment les uns des autres.

Le dispositif de contrôle de pression ou alternativement de force d'injection 1 comprend par ailleurs un support 11 portant solidairement chacun des éléments de mesure 10 de pression ou alternativement de force d'injection. Les éléments de mesure 10 de pression ou alternativement de force d'injection peuvent avantageusement être montés sur le support par vissage ou encore surmoulé sur celui-ci. Par exemple, le corps de l'élément de mesure 10 peut être fileté et une ouverture taraudée du support 11 peut être prévue pour recevoir l'élément de mesure 10.

Le support 11 des éléments de mesure 10 permet leur mise en position les uns par rapport aux autres de sorte qu'ils correspondent à un agencement permettant à chacun des éléments de mesure 10 de recevoir un jet correspondant.

On entend par « jet correspondant » ou « élément de mesure correspondant », un jet ou un élément de mesure associé respectivement à un élément de mesure ou à un jet.

Pour permettre la mesure de la pression ou alternativement de force d'injection de chaque jet, chaque élément de mesure 10 de pression ou alternativement de force d'injection du jet correspondant comprend une face de réception 10a sur laquelle est projeté le jet correspondant.

Pour limiter l'incidence d'un jet voisin sur la face de réception 10a d'un élément de mesure 10 correspondant, la face de réception 10a de chaque élément de mesure 10 est inclinée par rapport à un plan orthogonal à la direction d'injection du jet correspondant. L'inclinaison de chaque face de réception 10a est orientée distinctement l'une de l'autre.

Dans ce premier mode de réalisation, chaque élément de mesure 10 de pression ou de force d'injection est formé par l'assemblage d'un capteur 12 de pression ou alternativement de force d'injection et d'une pièce rapportée 13 comprenant la face de réception 10a du jet correspondant.

La pièce rapportée 13 est une pièce complémentaire au capteur 12 de pression ou alternativement de force. La face de réception 10a de l'élément de mesure 10 est une face en biseau de la pièce rapportée 13.

Dans ce premier mode de réalisation de l'invention, les faces de réception 10a, ici les faces en biseau, des éléments de mesure 10 sont chacune orientée radialement vers l'extérieur par rapport l'axe principal d'injection O. L'incidence d'un jet sur une face de réception 10a est alors projeté radialement vers l'extérieur par rapport l'axe principal d'injection O.

La pièce rapportée 13 est avantageusement prévue pour transmettre au capteur 12 de pression ou de force les efforts qu'elle reçoit sur sa face en biseau. Pour cela, à titre non limitatif, un élément de transmission mécanique peut être prévu pour relier mécaniquement l'une à l'autre, d'un côté, une face interne de la pièce rapportée 13 opposée à la face en biseau, et d'un autre côté, une face du capteur 12 de pression ou alternativement de force sur laquelle il est prévue d'appliquer une pression ou alternativement de force pour sa mesure. L'élément de transmission mécanique peut être formé par une lame, en forme de « Z », par exemple, dont les faces d'extrémités relient la face interne de la pièce rapportée 13 à la face du capteur 12 de pression ou alternativement de force, tel que défini précédemment.

Le capteur 12 de pression ou alternativement de force est avantageusement un capteur piézoélectrique sensible aux variations mécaniques de la face de réception 10a lorsque celle-ci reçoit le jet correspondant. Lorsqu'un jet est projeté depuis un canal sur la face de réception 10a de l'élément de mesure 10, la pression ou alternativement la force de ce jet peut alors être mesurée.

Le capteur 12 de pression ou alternativement de force peut avantageusement être un capteur de type crayon permettant sa mise en regard d'un canal 25a de la buse d'injection 25 indépendamment des autres éléments de mesure 10 de pression ou de force d'injection. En d'autres termes, dans le cas d'une pluralité de canaux 25a, les capteurs de type crayon de taille réduite peut être mis en regard des canaux 25a de la buse d'injection 25 sans être en contact les uns avec les autres. La mesure de la pression ou alternativement de la force d'injection de chacun des jets indépendamment les uns des autres se trouve alors améliorée.

La combinaison d'un capteur 12 de pression ou alternativement de force avec une pièce rapportée 13 comportant une face en biseau permet avantageusement d'étendre les capteurs 12 de pression ou alternativement de force d'injection dans leur longueur parallèlement à la direction d'injection du jet correspondant. Une telle configuration permet de faciliter l'assemblage des éléments de mesure 10 sur le support 11. Par ailleurs, la connectique du dispositif de contrôle de pression ou alternativement de force 1 s'en trouve facilité. En effet, une extrémité libre d'un capteur 12 peut être insérée dans une fiche en correspondance lorsque le dispositif de contrôle de pression ou alternativement de force d'injection 1 est logé dans un socle 4 d'un poste opérationnel.

Des ergots 14 d'indexage du support 11 sont prévus pour l'indexage des éléments de mesure 10 de pression ou alternativement de force d'injection. Plus particulièrement, chacun des ergots 14 d'indexage permet avantageusement l'indexage radiale et axiale d'une pièce rapportée 13 qui lui est associée. Chaque ergot 14 comprend un orifice radial 14a par rapport à l'axe principal d'injection O et recevant un doigt d'indexage 14b prévu pour venir indexer radialement et axialement chacune des pièces rapportées 13 et assurer avantageusement leurs orientations les unes par rapport aux autres.

On comprendra alors que l'indexage des pièces rapportées 13 par les ergots 14 d'indexage permet d'améliorer la mesure de pression ou alternativement de force des jets en limitant le rapport signal à bruit de l'information transmise par un élément de mesure 10 correspondant.

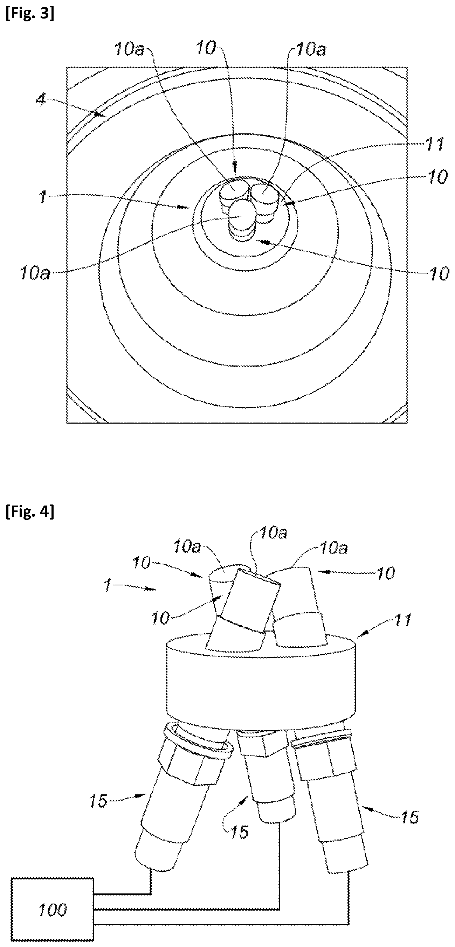

A la figure 4, on a représenté un deuxième mode de réalisation de l'invention dans lequel chaque élément de mesure 10 de pression ou alternativement de force d'injection est formé d'une seule pièce par un capteur 15 de pression.

Dans ce deuxième mode de réalisation, l'inclinaison de la face de réception 10a de chaque élément de mesure 10 de pression ou alternativement de force d'injection est obtenue par l'inclinaison dans sa longueur de l'élément de mesure 10 de pression ou alternativement de force d'injection correspondant par rapport à la direction d'injection O.

De façon similaire au premier mode de réalisation, le capteur 15 de pression ou alternativement de force peut être formé par un capteur piézoélectrique sensible aux variations mécaniques de la face de réception 10a lorsque celle-ci reçoit le jet correspondant.

Les éléments de mesure 10 peuvent être montés sur le support 11 de façon identique au premier mode de réalisation.

Le capteur 15 de pression ou alternativement de force peut également être de type crayon.

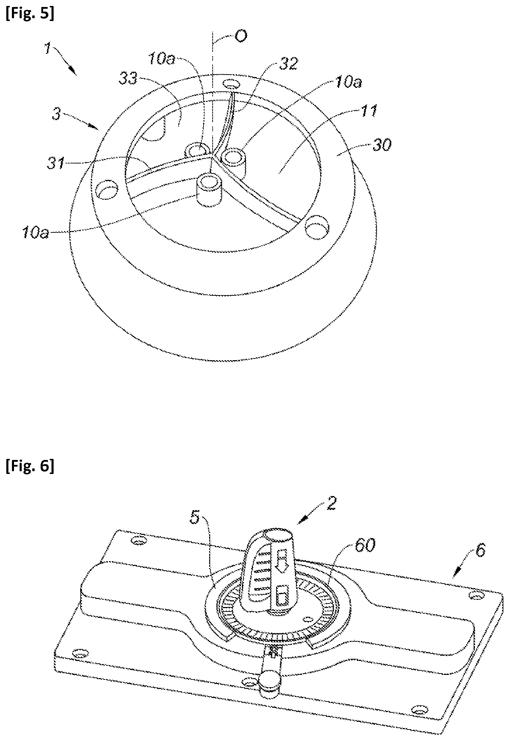

A la figure 5, on a représenté un troisième mode de réalisation de l'invention dans lequel les éléments de mesure 10 de pression ou alternativement de force d'injection présente chacun une face de réception 10a orthogonale à la direction d'injection O.

Dans ce troisième mode de réalisation et de façon combinable avec l'un quelconque des modes de réalisation de l'invention, il est avantageusement prévu un organe de cloisonnement 3 des jets les uns par rapport aux autres. L'organe de cloisonnement 3 des jets est formé par un contour circulaire 30 depuis lequel s'étendent radialement vers l'intérieur par rapport à l'axe principal d'injection O des séparateurs 31 reliés entre eux par une partie centrale 32 de l'organe de cloisonnement 3. L'organe de cloisonnement 3 est avantageusement monté sur des pieds 33 pour être surélevé par rapport au support 11 du dispositif de contrôle de pression ou alternativement de force d'injection 1. Lorsque l'organe de cloisonnement 3 est monté sur le support 11 ou sur le socle 4 d'un poste de travail, les séparateurs 31 viennent délimiter les éléments de mesure 10 les uns des autres. Plus particulièrement, les séparateurs 32 sont positionnés dans la direction de l'axe principal d'injection O pour dépasser des faces de réception 10a des éléments de mesure 10 tout en les délimitant. De cette façon, l'organe de cloisonnement 3 permet d'empêcher l'incidence d'un jet voisin sur la face de réception 10a d'un élément de mesure 10 correspondant.

On comprendra que l'organe de cloisonnement 3 des jets permet d'améliorer la mesure de chacun des jets.

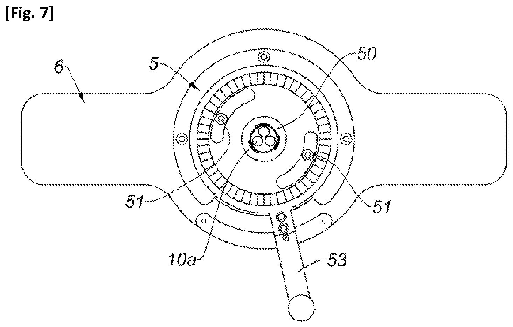

Aux figures 6 et 7, on a représenté un élément d'indexage 5 des canaux 25a de la buse d'injection 25 par rapport aux éléments de mesure 10 de pression ou de force d'injection.

Tel que représenté, l'élément d'indexage 5 est prévu pour être reçu de façon amovible dans une ouverture 60 d'un plateau 6. Sur un poste opérationnel, ce plateau 6 est positionné de sorte que l'ouverture 60 donne accès au dispositif de contrôle de pression ou alternativement de force d'injection 1.

L'élément d'indexage 5 comprend une base de réception 50 de la buse d'injection 25 et au moins deux points d'indexage 51 prévus pour assurer la mise en position de l'élément d'indexage 5 par rapport aux éléments de mesure 10 de pression ou alternativement de force d'injection.

Une manette 53 de l'élément d'indexage 5 permet de le manipuler en rotation par rapport à l'axe principal d'injection O, ceci pour atteindre une position finale de mise en correspondance des points d'indexation 51 avec une partie complémentaire du plateau 6.

Dans cette position finale, les canaux 25a de la buse d'injection 25 sont mis en regard des éléments de mesure 10 de pression ou alternativement de force d'injection.

On comprendra que l'élément d'indexage 5 assure une projection précise de chaque jet sur la face de réception 10a de l'élément de mesure 10 correspondant.

Le dispositif de contrôle de pression ou alternativement de force d'injection 1 permet la mise en œuvre d'un procédé de contrôle de pression ou alternativement de force d'injection d'une pluralité de jets générés par des canaux 25a en sortie d'une buse 25 d'un dispositif d'injection sans aiguille 2.

Le procédé est bien entendu réalisé à l'aide d'un dispositif de contrôle de pression ou alternativement de force d'injection 1 selon l'un quelconque des modes de réalisation de l'invention. Ce procédé de contrôle comprend au moins :
- une étape d'indexage dans laquelle les canaux 25a de la buse 25 du dispositif d'injection sans aiguille 2 sont mis en regard des éléments de mesure 10 de pression ou alternativement de force d'injection du dispositif de contrôle de pression ou alternativement de force d'injection 1 ;
- une étape de mesure de pression ou alternativement de force d'injection dans laquelle chacun des jets est mesuré indépendamment les uns des autres.

La mesure de ces jets est avantageusement réalisée par l'unité de traitement 100.

Bien évidemment, l'invention n'est pas limitée aux exemples qui viennent d'être décrits et de nombreux aménagements peuvent être apportés à ces exemples sans sortir du cadre de l'invention. Notamment, les différentes caractéristiques, formes, variantes et modes de réalisation de l'invention peuvent être associés les uns avec les autres selon diverses combinaisons dans la mesure où ils ne sont pas incompatibles ou exclusifs les uns des autres. En particulier, toutes les variantes et modes de réalisation décrits précédemment sont combinables entre eux.

## Revendications

1. Dispositif de contrôle de pression ou alternativement de force d'injection (1) d'une pluralité de jets générés par des canaux (25a) en sortie d'une buse (25) d'un dispositif d'injection sans aiguille (2), le dispositif de contrôle de pression ou alternativement de force d'injection (1) comprenant pour chaque jet généré en sortie de la buse (25) un élément de mesure (10) de pression ou alternativement de force d'injection du jet correspondant, chaque élément de mesure (10) de pression ou alternativement de force d'injection comprenant une face de réception (10a) du jet correspondant orientée pour recevoir ce jet.

2. Dispositif de contrôle de pression ou alternativement de force (1) selon la revendication précédente, **caractérisé en ce qu'**au moins la face de réception (10a) d'un élément de mesure (10) de pression ou alternativement de force d'injection est inclinée par rapport à un plan orthogonal à la direction de propagation du jet correspondant.

3. Dispositif de contrôle de pression ou alternativement de force (1) selon la revendication précédente, **caractérisé en ce que** l'inclinaison de la face de réception (10a) d'au moins un élément de mesure (10) de pression ou alternativement de force d'injection est obtenue par l'inclinaison dans sa longueur de l'élément de mesure (10) de pression ou alternativement de force d'injection correspondant par rapport audit plan orthogonal à la direction de propagation.

4. Dispositif de contrôle de pression ou alternativement de force (1) selon l'une des revendications précédentes, **caractérisé en ce qu'**au moins un élément de mesure (10) de pression ou alternativement de force d'injection est formé par l'assemblage d'un capteur (15) de pression ou alternativement de force d'injection et d'une pièce rapportée (13) comprenant la face de réception (10a) du jet correspondant.

5. Dispositif de contrôle de pression ou alternativement de force (1) selon l'une quelconque des revendications précédentes, **caractérisé en ce qu'**il comprend un support (11) portant solidairement chacun des éléments de mesure (10) de pression ou alternativement de force d'injection.

6. Dispositif de contrôle de pression ou alternativement de force (1) selon la revendication précédente, **caractérisé en ce que** le support (11) comprend des ergots (14) d'indexage des éléments de mesure (10) de pression ou alternativement de force d'injection.

7. Dispositif de contrôle de pression ou alternativement de force (1) selon l'une quelconque des revendications précédentes, **caractérisé en ce qu'**il comprend un organe de cloisonnement (3) des jets les uns par rapport aux autres.

8. Dispositif de contrôle de pression ou alternativement de force (1) selon la revendication précédente, **caractérisé en ce que** l'organe de cloisonnement (3) des jets comprend des séparateurs (32) délimitant les éléments de mesure (10) de pression ou alternativement de force d'injection les uns des autres en vue d'empêcher l'incidence d'un jet voisin sur la face de réception (10a) d'un élément de mesure (10) de pression ou alternativement de force d'injection correspondant.

9. Dispositif de contrôle de pression ou alternativement de force (1) selon l'une quelconque des revendications précédentes, **caractérisé en ce qu'**il comprend un élément d'indexage (5) des canaux (25a) de la buse (25) par rapport aux éléments de mesure (10) de pression ou alternativement de force d'injection.

10. Dispositif de contrôle de pression ou alternativement de force (1) selon la revendication précédente, **caractérisé en ce que** l'élément d'indexage (5) comprend une base de réception (50) de la buse (25) et au moins deux points d'indexage (51) prévus pour assurer la mise en position de l'élément d'indexage (5) par rapport aux éléments de mesure (10) de pression ou alternativement de force d'injection.

11. Ensemble de contrôle de pression ou alternativement de force d'injection comprenant un dispositif d'injection sans aiguille (2) et un dispositif de contrôle de pression ou alternativement de force d'injection (1) selon l'une quelconque des revendications précédentes.

12. Procédé de contrôle de pression ou alternativement de force d'injection d'une pluralité de jets générés par des canaux (25a) en sortie d'une buse (25) d'un dispositif d'injection sans aiguille (2), le procédé étant réalisé à l'aide d'un dispositif de contrôle de pression ou alternativement de force (1) selon l'une quelconque des revendications 1 à 10,
le procédé de contrôle étant **caractérisé en ce qu'**il comprend :
- une étape d'indexage dans laquelle les canaux (25a) de la buse (25) du dispositif d'injection sans aiguille (2) sont mis en regard des éléments de mesure de force d'injection du dispositif de contrôle de pression ou alternativement de force (1) d'injection ;
- une étape de mesure de pression ou alternativement de force d'injection dans laquelle chacun des jets est mesuré indépendamment les uns des autres.

## Patentansprüche

1. Kontrollvorrichtung für Druck oder alternativ Injektionskraft (1) einer Vielzahl von Strahlen, die durch Kanäle (25a) am Ausgang einer Düse (25) einer nadellosen Injektionsvorrichtung (2) erzeugten Strahlen, wobei die Kontrollvorrichtung für Druck oder alternativ Injektionskraft (1) für jeden am Ausgang der Düse (25) erzeugten Strahl ein Druck- oder alternativ Injektionskraft-Messelement (10) des entsprechenden Strahls umfasst, wobei jedes Druck- oder alternativ Injektionskraft-Messelement (10) eine Aufnahmefläche (10a) des entsprechenden Strahls umfasst, die ausgerichtet ist, um diesen Strahl aufzunehmen.

2. Kontrollvorrichtung für Druck oder alternativ Kraft (1) nach dem vorstehenden Anspruch, **dadurch gekennzeichnet, dass** mindestens die Aufnahmefläche (10a) eines Druck- oder alternativ Injektionskraft-Messelements (10) in Bezug auf eine Ebene orthogonal zur Ausbreitungsrichtung des entsprechenden Strahls geneigt ist.

3. Kontrollvorrichtung für Druck oder alternativ Kraft (1) nach dem vorstehenden Anspruch, **dadurch gekennzeichnet, dass** die Neigung der Aufnahmefläche (10a) mindestens eines Druck- oder alternativ Injektionskraft-Messelements (10) durch die Neigung in seiner Länge des entsprechenden Druck- oder alternativ Injektionskraft-Messelements (10) in Bezug auf die genannte Ebene orthogonal zur Ausbreitungsrichtung erhalten wird.

4. Kontrollvorrichtung für Druck oder alternativ Kraft (1) nach einem der vorstehenden Ansprüche, **dadurch gekennzeichnet, dass** mindestens ein Druck- oder alternativ Injektionskraft-Messelements (10) durch das Zusammensetzen eines Druck- oder alternativ Injektionskraftsensors (15) und eines Anbauteils (13) gebildet wird, das die Aufnahmefläche (10a) des entsprechenden Strahls umfasst.

5. Kontrollvorrichtung für Druck oder alternativ Kraft (1) nach einem der vorstehenden Ansprüche, **dadurch gekennzeichnet, dass** sie einen Halter (11) umfasst, der jedes der Druck- oder alternativ Injektionskraft-Messelemente (10) fest trägt.

6. Kontrollvorrichtung für Druck oder alternativ Kraft (1) nach dem vorstehenden Anspruch, **dadurch gekennzeichnet, dass** der Halter (11) Rastnasen (14) zur Indexierung der Druck- oder alternativ Injektionskraft-Messelemente (10) umfasst.

7. Kontrollvorrichtung für Druck oder alternativ Kraft (1) nach einem der vorstehenden Ansprüche, **dadurch gekennzeichnet, dass** sie ein Trennelement (3) der Strahlen zueinander umfasst.

8. Kontrollvorrichtung für Druck oder alternativ Kraft (1) nach dem vorstehenden Anspruch, **dadurch gekennzeichnet, dass** das Trennelement (3) der Strahlen Separatoren (32) umfasst, welche die Druck- oder alternativ Injektionskraft-Messelemente (10) in Hinblick darauf voneinander abgrenzen, das Auftreffen eines benachbarten Strahls auf die Aufnahmefläche (10a) eines entsprechenden Druck- oder alternativ Injektionskraft-Messelements (10) zu verhindern.

9. Kontrollvorrichtung für Druck oder alternativ Kraft (1) nach einem der vorstehenden Ansprüche, **dadurch gekennzeichnet, dass** sie ein Element zur Indexierung (5) der Kanäle (25a) der Düse (25) in Bezug auf die Druck- oder alternativ Injektionskraft-Messelemente (10) umfasst.

10. Kontrollvorrichtung für Druck oder alternativ Kraft (1) nach dem vorstehenden Anspruch, **dadurch gekennzeichnet, dass** das Element zur Indexierung (5) eine Aufnahmebasis (50) der Düse (25) und mindestens zwei Indexierungspunkte (51) umfasst, die vorgesehen sind, um die Positionierung des Elements zur Indexierung (5) in Bezug auf die Druck- oder alternativ Injektionskraft-Messelemente (10) sicherzustellen.

11. Einheit zur Kontrolle von Druck oder alternativ Injektionskraft, umfassend eine nadellose Injektionsvorrichtung (2) und eine Kontrollvorrichtung für Druck oder alternativ Kraft (1) nach einem der vorstehenden Ansprüche.

12. Verfahren zur Kontrolle von Druck oder alternativ Injektionskraft einer Vielzahl von Strahlen, die durch Kanäle (25a) am Ausgang einer Düse (25) einer nadellosen Injektionsvorrichtung (2) erzeugt werden, wobei das Verfahren mithilfe einer Kontrollvorrichtung für Druck oder alternativ Kraft (1) nach einem der Ansprüche 1 bis 10 durchgeführt wird,
wobei das Kontrollverfahren **dadurch gekennzeichnet ist, dass** es umfasst:
- einen Indexierungsschritt, bei dem die Kanäle (25a) der Düse (25) der nadellosen Injektionsvorrichtung (2) gegenüber den Injektionskraftmesselementen der Kontrollvorrichtung für Druck oder alternativ Injektionskraft (1) angeordnet sind,
- einen Messschritt für Druck oder alternativ Injektionskraft, bei dem jeder der Strahlen unabhängig voneinander gemessen wird.

## Claims

1. A device (1) for controlling the injection pressure or alternatively force of a plurality of jets generated by channels (25a) at the outlet of a nozzle (25) of a needleless injection device (2), the injection pressure or alternatively force control device (1) comprising, for each jet generated at the outlet of the nozzle (25), an element (10) for measuring the injection pressure or alternatively force of the corresponding jet, each injection pressure or alternatively force measuring element (10) comprising a receiving face (10a) for the corresponding jet oriented to receive this jet.

2. The pressure or alternatively force control device (1) according to the preceding claim, **characterized in that** at least the receiving face (10a) of an injection pressure or alternatively force measuring element (10) is inclined relative to a plane orthogonal to the direction of propagation of the corresponding jet.

3. The pressure or alternatively force control device (1) according to the preceding claim, **characterized in that** the inclination of the receiving face (10a) of at least one injection pressure or alternatively force measuring element (10) is obtained by the inclination along its length of the corresponding injection pressure or alternatively force measuring element (10) relative to said plane orthogonal to the direction of propagation.

4. The pressure or alternatively force control device (1) according to any of the preceding claims, **characterized in that** at least one injection pressure or alternatively force measuring element (10) is formed by the assembly of an injection pressure or alternatively force sensor (15) and an insert (13) comprising the receiving face (10a) for the corresponding jet.

5. The pressure or alternatively force control device (1) according to any one of the preceding claims, **characterized in that** it comprises a support (11) integrally carrying each of the injection pressure or alternatively force measuring elements (10).

6. The pressure or alternatively force control device (1) according to the preceding claim, **characterized in that** the support (11) comprises lugs (14) for indexing the injection pressure or alternatively force measuring elements (10).

7. The pressure or alternatively force control device (1) according to any one of the preceding claims, **characterized in that** it comprises a member (3) for partitioning the jets relative to each other.

8. The pressure or alternatively force control device (1) according to the preceding claim, **characterized in that** the jet partitioning member (3) comprises separators (32) delimiting the injection pressure or alternatively force measuring elements (10) from each other in order to prevent the impact of a neighboring jet on the receiving face (10a) of a corresponding injection pressure or alternatively force measuring element (10).

9. The pressure or alternatively force control device (1) according to any one of the preceding claims, **characterized in that** it comprises an element (5) for indexing the channels (25a) of the nozzle (25) relative to the injection pressure or alternatively force measuring elements (10).

10. The pressure or alternatively force control device (1) according to the preceding claim, **characterized in that** the indexing element (5) comprises a receiving base (50) of the nozzle (25) and at least two indexing points (51) provided to ensure the positioning of the indexing element (5) relative to the injection pressure or alternatively force measuring elements (10).

11. An injection pressure or alternatively force control assembly comprising a needleless injection device (2) and an injection pressure or alternatively force control device (1) according to any one of the preceding claims.

12. A method for controlling the injection pressure or alternatively force of a plurality of jets generated by channels (25a) at the outlet of a nozzle (25) of a needleless injection device (2), the method being carried out using a pressure or alternatively force control device (1) according to any one of claims 1 to 10,
the control method being **characterized in that** it comprises:
- an indexing step in which the channels (25a) of the nozzle (25) of the needleless injection device (2) are placed opposite the injection force measuring elements of the injection pressure or alternatively force control device (1);
- an injection pressure or alternatively force measuring step in which each of the jets is measured independently of each other.
